**Europäisches Patentamt**

**European Patent Office**

**Office européen des brevets**

(19)

(11) Veröffentlichungsnummer: **0 174 460**
**B1**

(12) **EUROPÄISCHE PATENTSCHRIFT**

(45) Veröffentlichungstag der Patentschrift:
**17.11.88**

(51) Int. Cl.⁴: **A 61 B 6/04**

(21) Anmeldenummer: **85108662.9**

(22) Anmeldetag: **11.07.85**

(54) **Patiententisch.**

(30) Priorität: **14.08.84 CH 3904/84**

(43) Veröffentlichungstag der Anmeldung:
**19.03.86 Patentblatt 86/12**

(45) Bekanntmachung des Hinweises auf die Patenterteilung:
**17.11.88 Patentblatt 88/46**

(84) Benannte Vertragsstaaten:
**DE FR GB IT NL SE**

(56) Entgegenhaltungen:
**EP - A - 0 069 607**
**EP - A - 0 077 447**
**EP - A - 0 110 475**
**FR - A - 2 115 423**
**US - A - 3 428 307**

(73) Patentinhaber: **BBC Brown Boveri AG, Haselstrasse, CH-5401 Baden (CH)**

(72) Erfinder: **Schär, Hugo, Im Tuech, CH-8416 Flaach (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft einen Patiententisch gemäss Oberbegriff des Patentanspruches 1.

Aus der Druckschrift EP-A3-0 077 447 ist ein Patiententisch bekannt, bei welchem eine Liegefläche auf einer Säule in Längsrichtung verschiebbar angebracht ist. Um die Liegefläche weit über die tragende Säule hinausschieben zu können, ist ein Zwischenträger zwischen Säule und Liegeplatte angeordnet.

Die Forderung nach einer Verschiebbarkeit der Liegefläche des Tisches sowohl längs wie quer, wobei die Längsverschiebung zudem beidseitig weit über die die Liegefläche tragende Säule hinausreichen muss, ergibt bei den bekannten Tischen eine konstruktiv bedingte sehr grosse Dicke bzw. Höhe des Tisches. Dadurch wird zwangsläufig der Hubbereich nach unten (minimale Höhe) sehr stark eingeschränkt.

Ein weiterer Nachteil der bekannten Tische besteht in der zu geringen Stabilität der Liegefläche in den Extremstellungen, d.h. voll ausgefahren bezüglich der Tragsäule.

Der im Anspruch 1 angegebenen Erfindung liegt die Aufgabe zugrunde, einen Patiententisch zu schaffen, be dem die Bauhöhe der Liegefläche mit den Führungen für die Quer- und Längsverschiebung vermindert und dessen Stabilität bezüglich der Abstützung der Momente bei ausgefahrener Liegeplatte trotz verminderter Bauhöhe verbessert werden kann.

Diese Aufgaben werden durch die im kennzeichnenden Teil des Patentanspruches 1 angegebene Erfindung gelöst.

Es ist nun möglich, durch die Verwendung eines nur einseitig angebrachten Zwischenträgers alle Führungen für die Längsverschiebung des Tisches in einem Raum mit geringer Bauhöhe anzuordnen.

Vorteilhafte Weiterbildungen der Erfindung sind in den abhängigen Ansprüchen beschrieben.

Bei einer vorteilhaften Ausgestaltung der Erfindung besteht der Zwischenträger aus zwei je zwei Nuten aufweisenden Stahlprofilen, zwischen denen ein endloses Band um zwei Rollen umlaufend eingesetzt ist, mittels welchem die Liegeplatte antreibbar ist. In ausgefahrener Stellung werden durch den Zwischenträger eine lange Basis für die durch die Liegeplatte und den Patienten erzeugten Momente gebildet.

Durch die stromleitende Ausbildung des Bandes bzw. eine auf das Band aufgebrachte Kupferfolie kann eine zuverlässige Erdung der Liegeplatte gewährleistet werden.

In einer weiteren vorteilhaften Ausgestaltung der Erfindung sind die als Gegenstück zu den Nuten im Zwischenträger notwendigen Nuten an Rippen am Führungssockel und am Fortsatz der Liegeplatte angebracht.

Eine spielfreie und damit auch erschütterungsfreie Führung bilden Zylinderrollen mit abwechslungsweise mit um 90° zueinander liegenden Achsen.

In vorteilhafter Weise wird die Liegeplatte von einem Antriebsmotor angetrieben, dessen Abtriebsritzel im Eingrfff mit einer Verzahnung am Zwischenträger steht.

Dank der seitlichen Anordnung des Zwischenträgers kann einerseits die Bauhöhe gering gehalten werden und zudem ein hoher Steg unter der Liegeplatte vorgesehen werden, welcher sich günstig auf die Durchbiegung auswirkt.

Anhand eines illustrierten Ausführungsbeispieles wird die Erfindung näher beschrieben. Es zeigen:

Figur 1 einen Querschnitt durch einen Patiententisch längs Linie I–I in Figur 2, teilweise in Ansicht,

Figur 2 einen Grundriss des Patiententisches,

Figur 3 einen Querschnitt durch die Liegefläche längs Linie III–III in Figur 2,

Figur 4 einen Schnitt längs Linie IV–IV in Figur 3,

Figur 5 einen Querschnitt durch die Liegefläche längs Linie III–III in Figur 2,

Figur 6 einen Schnitt längs Linie VI–VI in Figur 5.

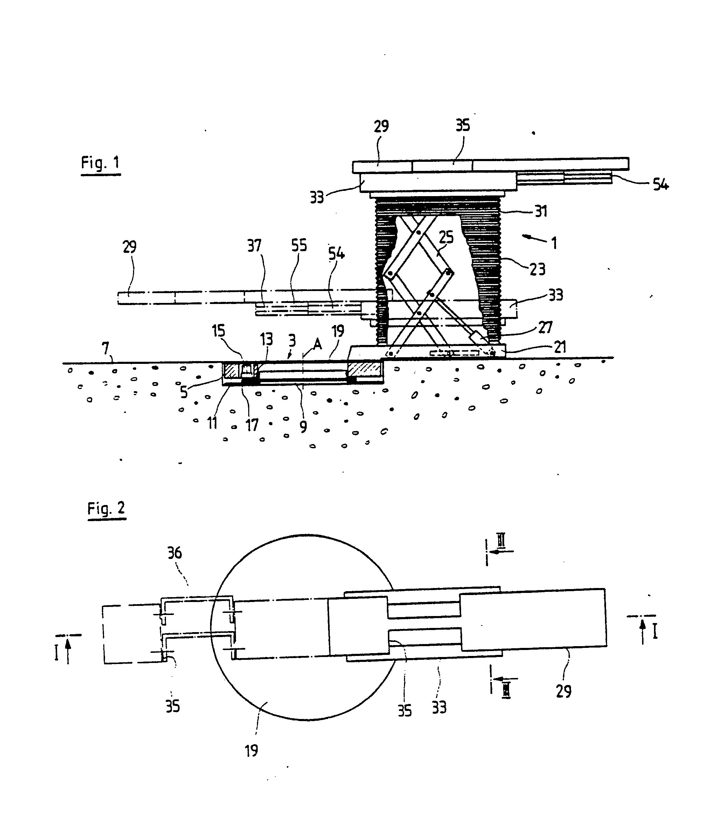

Auf einer Drehachse A ist in Figur 1 ein Patiententisch 1 drehbar gelagert. Das Drehlager 3, welches nicht Teil der Erfindung ist und nur zum besseren Verständnis kurz beschrieben wird, besteht aus einem in einer Vertiefung 5 des Bodens 7 fest eingesetzten Zahnkranz 9 sowie einem Ring 11, an dessen Innenseite ein Kugel- oder Rollenlager 13 aufgesetzt und am Zahnkranz 9 abgestützt ist.

Am Ring 11 ist eine Motorgetriebeeinheit 15, z.B. ein herkömmlicher Gleichstrommotor mit einem Schneckengetriebe, mit einem Antriebsritzel 17 befestigt und in Antriebsverbindung mit dem Zahnkranz 9.

Auf dem Ring 11, der von einer scheibenförmigen Platte 19 überdeckt ist, ist der Fuss 21 der Säule 23 des Tisches 1 aufgesetzt. In der Säule 23 befindet sich eine Hubschere 25, welche von einem hydraulisch betriebenen oder elektrisch betriebenen Antrieb 27 von einer gefalteten Stellung, in welcher sich die Liegeplatte 29 des Tisches 1 in abgesenkter Lage befindet, in eine gestreckte Stellung (Tisch 1 angehoben) verfahrbar ist. Zum Schutz der Hubschere 25 sowie deren Gelenke und Führungen sowie als Unfallschutz ist die Säule 23 von einem Faltenbalg 31 umschlossen.

Die Liegeplatte 29 ist in einem Führungssockel 23 längsverschiebbar gelagert. Der Führungssockel 33 ist zusammen mit der Liegeplatte 29 zudem quer zur Längsausdehnung der Liegeplatte 29 auf der Säule 23 verschiebbar. Die Ausbildung der Querführung ist nicht Teil der Erfindung und deshalb auch nicht näher gezeigt und beschrieben; sie kann ähnlich oder gleich ausgeführt sein wie die Längsführung. In der Liegeplatte 29 können seitlich je eine Ausnehmung 35 zum Anbringen von sogenannten C-Armen 36, welche – nach aussen gelegt (oben) – eine Bestrahlung von unten ermöglichen oder – nach innen gelegt (unten) – eine seitliche Bestrahlung ermöglichen, vorgesehen sein (Figur 2).

In den Figuren 1 und 2 sind die Extremstellungen der Liegeplatte 29 sichtbar; in ausgezogenen Linien ist die Liegeplatte einerseits in der höchst möglichen Höhe und nach rechts ausgefahren; in strichpunktierten Linien ist die Liegeplatte 29 in tiefster Stellung und vollständig nach links ausgefahren.

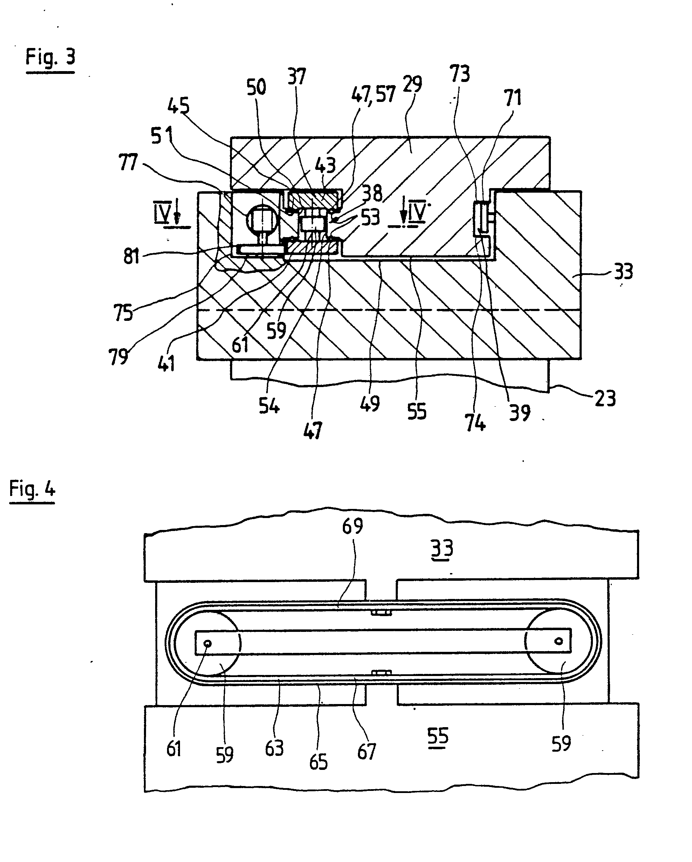

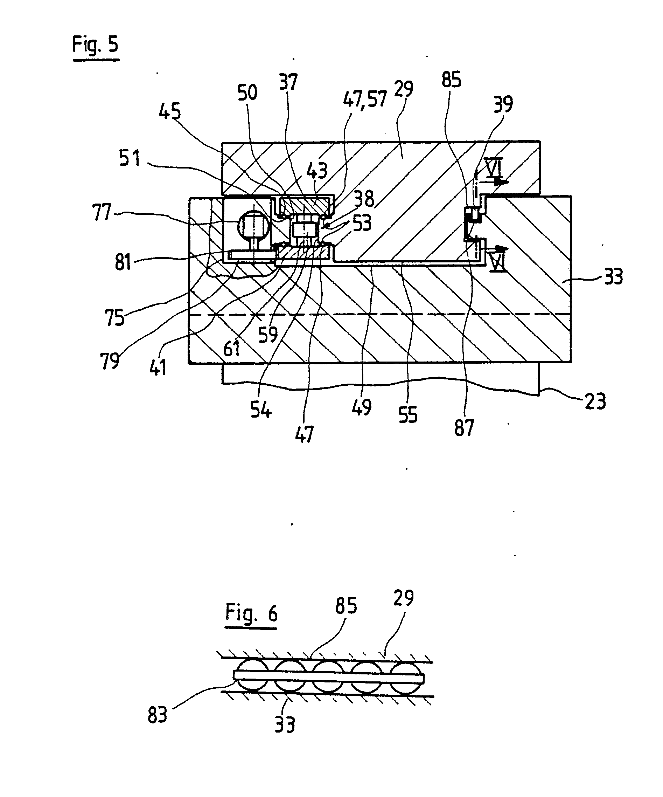

Im Schnitt nach der Figur 3 sind die Führungen 37 und 39 für die Längsverschiebung der Liegeplatte 29 im Detail sichtbar. Die Führung 37 besteht aus zwei parallel zueinander und in einem Abstand liegenden und fest miteinander verbundenen, einen Zwischenträger 38 bildenden Stahlprofilen 41, 43, welche an den einander zugekehrten Seiten V-förmige Nuten 45, 47 aufweisen. Als Gegenstück zu den Nuten 45 am Profil 43 sind in einer Ausnehmung 49 am Führungssockel 33 längs der Ausnehmung 49 auf einer Rippe 50 verlaufende Nuten 51 angebracht. Den Nuten 47 liegen analog Nuten 53 gegenüber, welche an einem in die Ausnehmung 49 eingreifenden und mit einer Rippe 54 versehenen Steg 55 der Liegeplatte 29 eingelassen sind. In den Nuten 45, 51 und 47, 53 sind mit wechselnder Achslage Zylinderrollen 57 eingelegt, z.B. Längsführungen vom Typ RD der Firma Schneeberger in Roggwil, Schweiz, so dass eine reibungsarme und präzise Längsführung der Liegeplatte 29 auf dem Führungssockel 33 gewährleistet ist. Anstelle V-förmiger Nuten können auch Nuten mit kreisbogenförmigem Querschnitt und Kugeln vorgesehen werden. Der aus den Profilen 41 und 43 bestehende Zwischenträger 38 weist an den Enden der Profile 41 und 43 angebrachte Umlenkrollen 59 auf, die an vertikal angebrachten Achsen 61 drehbar gelagert sind (vgl. auch Figur 4). Über die Umlenkrollen 59 läuft ein endloses Band 63, welches entweder mit einem Stromleiter 65 beschichtet ist oder selbst stromleitend ausgebildet ist, um eine elektrische Verbindung zwischen der Liegeplatte 29 und dem Führungssockel 33 herzustellen. Das Band 63 ist am einen Trum 67 mit dem Steg 55 der Liegeplatte 29 und am anderen Trum 69 mit dem Führungssockel 33 verschraubt, so dass eine mechanische Verbindung zwischen der Liegeplatte 29 und dem Führungssockel 33 hergestellt ist. Die Höhe des Zwischenträgers 38 ist vorzugsweise gleich oder etwas geringer als die Höhe des Steges 55.

Die Führung 39, die an der anderen Flanke des Steges 55 angeordnet ist, besteht aus einer Mehrzahl auf einer Geraden seitlich an der Ausnehmung 49 angeordneten Rollen 71, welche in eine am Steg 55 der Liegeplatte 29 angebrachte Nut 73 eingreifen und auf deren Flanken 74 abrollen können. In eine Ausnehmung 75 seitlich des Stahlprofiles 43 ist ein Antriebsmotor 77 mit einem Ritzel 79 eingesetzt, welches Ritzel 79 mit einer Verzahnung 81 am Profil 41 kämmt und dieses längs dem Führungssockel 33 verschieben kann. Als Antriebsmotor kann beispielsweise ein Flachmotor mit Getriebe vom Typ 0130 820 072 von Bosch Deutschland, verwendet werden.

In einer anderen Ausgestaltung der Erfindung gemäss Figuren 5/6 tritt anstelle der Rollen 71 eine Rollenführung 83, z.B. ein Kugelkäfig Typ JJ der Firma Schneeberger in Roggwil, Schweiz, welche zwischen der Abrollfläche 85 an der Liegeplatte 29 und einer Abrollfläche 87 am Führungssockel 33 eingelegt ist.

Durch die Verbindung der Liegeplatte 29 mit dem Führungssockel 33 über das Band 63 verschiebt sich die Liegeplatte 29 doppelt so schnell bzw. doppelt so weit wie die Führung 37, wenn diese durch den Antriebsmotor 77 angetrieben wird.

Zum Verschieben der Liegeplatte 29 in die linke Extremlage, d.h. vollständig nach links ausgefahren (Figur 1), wird durch den Antriebsmotor 77 die Führung 37 mit den Stahlprofilen 41 und 43 nach links bewegt. Weil der Motor 77 fest mit dem Führungssockel 33 verbunden ist und die Führung 37 durch das erste Trum 67 des endlosen Bandes 63 ebenfalls in Verbindung mit dem Führungssockel 33 steht und zudem das zweite Trum 69 an der Liegeplatte 29 angeschraubt ist, bewegt sich letztere mit doppelter Geschwindigkeit bzw. die Liegeplatte 29 legt immer den doppelten Weg wie die Führung 37 zurück. Bei vollständig ausgefahrener Liegeplatte 29 befindet sich die Führung 37, deren Länge etwa der Länge des Führungssockels 33 entspricht, je zur Hälfte innerhalb und ausserhalb des Führungssockels 33. Sie bildet damit sowohl eine exakte Führung für die Liegeplatte 29 als eine lange Basis für die Aufnahme des bei ausgefahrener und von einem Patienten belasteter Liegeplatte 29 entstehenden Momentes auf die Auflagepunkte zwischen der Liegeplatte 29 und dem Führungssockel 33.

**Patentansprüche**

1. Patiententisch mit einer Liegeplatte, welche in mindestens einer Richtung auf einem Führungssockel horizontal verschiebbar gelagert ist, wobei zur Erhöhung der Stabilität der Liegeplatte zwei im Abstand seitlich angebrachte Führungen und ein Zwischenträger zwischen Liegeplatte und Führungssockel vorgesehen sind, dadurch gekennzeichnet, dass nur eine Führung einen Zwischenträger (38) aufweist, der seitlich zwischen einem in den Führungssockel (33) versenkten Steg (55) der Liegeplatte (29) und dem Führungssockel (33) angeordnet ist.

2. Patiententisch nach Anspruch 1, dadurch gekennzeichnet, dass der Zwischenträger (38) aus zwei Stahlprofilen (41, 43) besteht, zwischen denen ein endloses Band (63) um zwei senkrecht zur Liegeplatte (29) angebrachte Achsen (61) drehbare Rollen (59) umlaufend angeordnet ist, welches Band (63) zwei Trume (67, 69) aufweist, welche parallel zum Zwischenträger (38) liegen, wobei das Band (63) am ersten Trum (67) mit dem Führungssockel (33) und am zweiten Trum (69) mit der Liegeplatte (29) verbunden ist.

3. Patiententisch nach Anspruch 2, dadurch gekennzeichnet, dass das Band (63) als Stromleiter ausgebildet ist.

4. Patiententisch nach Anspruch 3, dadurch gekennzeichnet, dass als Stromleiter ein Kupferband (65) auf das Band (63) aufgebracht ist.

5. Patiententisch nach Anspruch 2, dadurch gekennzeichnet, dass die zwei Stahlprofile (41, 43) an den einander zugekehrten Seiten je zwei Nuten (45, 47) aufweisen und dass zwischen die Nuten (45, 47) am Zwischenträger (38) je eine zwei Nuten (51, 53) aufweisende Rippe (50, 54) eingreift, wobei die eine Rippe (50) Teil des Führungssockels (33) und die Rippe (54) Teil des Steges (55) an der Liegeplatte (29) ist.

6. Patiententisch nach Anspruch 2, dadurch gekennzeichnet, dass zwischen den Nuten (45, 47) und (51, 53) Zylinderrollen (57) im wesentlichen spielfrei und abwechslungsweise mit um 90° zueinander liegenden Achsen eingelegt sind.

7. Patiententisch nach einem der Ansprüche 2 bis 6, dadurch gekennzeichnet, dass an einem der Stahlprofile (41, 43) eine Verzahnung (81) angebracht ist, in welche ein Ritzel (79) eines Antriebsmotors (77) eingreift.

8. Patiententisch nach Anspruch 7, dadurch gekennzeichnet, dass die Höhe des Zwischenträgers (38) geringer oder gleich der Höhe des Steges (55) ist.

9. Patiententisch nach Anspruch 8, dadurch gekennzeichnet, dass er sowohl in Längs- als auch in Querrichtung verschiebbar ist.

**Revendications**

1. Table pour patient avec un plateau d'examen qui peut coulisser horizontalement dans au moin une direction sur un socle de guidage, dans laquelle il est prévu, pour augmenter la stabilité du plateau d'examen, deux guides espacés latéralement l'un de l'autre et un appui intermédiaire entre le plateau d'examen et le socle de guidage, caractérisée en ce qu'un seul des guides présente un appui intermédiaire (38) qui est disposé latéralement entre une entretoise (55) du plateau d'examen (29) plongeant dans le socle de guidage (33) et le socle de guidage (33).

2. Table pour patient suivant la revendication 1, caractérisée en ce que l'appui intermédiaire (38) se compose de deux profilés en acier (41, 43) entre lesquels est disposée une bande sans fin (63) tournant autour de deux rouleaux rotatifs (59) dont les axes (61) sont perpendiculaires au plateau d'examen (29), laquelle bande (63) présente deux brins (67, 69) qui sont parallèles à l'appui intermédiaire (38), la bande (63) étant attachée au socle de guidage (33) par son premier brin (67) et au plateau d'examen (29) par son second brin (69).

3. Table pour patient suivant la revendication 2, caractérisée en ce que la bande (63) est elle-même conductrice de l'électricité.

4. Table pour patient suivant la revendication 3, caractérisée en ce qu'une bande de cuivre (65) est déposée sur la bande (3) en guise de conducteur de l'électricité.

5. Table pour patient suivant la revendication 2, caractérisée en ce que les deux profilés en acier (41, 43) présentent dans leurs faces opposés chacun deux rainures (45, 47) et en ce qu'entre les rainures (45, 47) de l'appui intermédiaire (38) s'engage une nervure (50, 54) présentant aussi deux rainures (51, 53), une des nervures (50) faisant partie du socle de guidage (33) et l'autre nervure (54) faisant partie de l'entretoise (55) du plateau d'examen (29).

6. Table pour patient suivant la revendication 2, caractérisée en ce qu'entre les rainures (45, 47) et (51, 53) sont posés des rouleaux cylindriques (57) essentiellement sans jeu et ayant en alternance des axes orientés à 90° l'un par rapport à l'autre.

7. Table pour patient suivant l'une ou l'autre des revendications 2 à 6, caractérisée en ce qu'un des profilés en acier (41, 43) est pourvu d'une denture (81) avec laquelle engrène un pignon (79) d'un moteur d'entraînement (77).

8. Table pour patient suivant la revendication 7, caractérisée en ce que la hauteur de l'appui intermédiaire (38) est inférieure ou égale à la hauteur de l'entretoise (55).

9. Table pour patient suivant la revendication 8, caractérisée en ce qu'elle peut coulisser aussi bien en direction longitudinale qu'en direction transversale.

**Claims**

1. Patient's table with a couch plate mounted horizontally slidably in at least one direction on a guide pedestal, two guides fitted laterally at an interval, and an intermediate support between couch plate and guide pedestal being provided for increasing the stability of the couch plate, characterised in that only one guide has an intermediate support (38), which is arranged laterally between a bar (55) of the couch plate (29), countersunk into the guide pedestal (33), and the guide pedestal (33).

2. Patient's table according to Claim 1, characterised in that the intermediate support (38) consists of two steel profiles (41, 43), between which a continuous belt (63) is arranged revolving round two rollers (59) rotatable about two axes (61) attached vertically to the couch plate (29), which belt (63) exhibits two sides (67, 69) which are located parallel to the intermediate support (38), the belt (63) being connected on the first side (67) to the guide pedestal (33) and on the second side (69) to the couch plate (29).

3. Patient's table according to Claim 2, characterised in that the belt (63) is constructed as an electric conductor.

4. Patient's table according to Claim 3, characterised in that a copper belt (65) is attached to the belt (63) as an electric conductor.

5. Patient's table according to Claim 2, characterised in that the two steel profiles (41, 43) each exhibit on the mutually facing sides two grooves (45, 47) and in that a rib (50, 54) exhibiting two grooves (51, 53) engages respectively between the grooves (45, 47) on the intermediate support (38), the one rib (50) being a part of the guide pedestal (33) and the rib (54) being a part of a bar (55) on the couch plate (29).

6. Patient's table according to Claim 2, characterised in that cylindrical rollers (57) are inserted substantially free from play and alternately with axes located mutually at 90° between the grooves (45, 47) and (51, 53).

7. Patient's table according to any of Claims 2 to 6, characterised in that a tooth system (81), with which a pinion (79 of a drive motor (77) meshes, is attached to one of the steel profiles (41, 43).

8. Patient's table according to Claim 7, characterised in that the height of the intermediate support (38) is less than or equal to the height of the bar (55).

9. Patient's table according to Claim 8, characterised in that it is slidable both in the longitudinal and transverse direction.

Fig. 1

Fig. 2

Fig. 3

Fig. 4

Fig. 5

Fig. 6